# EUROPEAN PATENT SPECIFICATION

(11) **EP 4 173 657 B1**
(45) Date of publication and mention of the grant of the patent: **12.11.2025**
(21) Application number: 21831791.5
(22) Date of filing: 11.06.2021
(51) Int. Cl.: A61M 5/303

(54) **NEEDLELESS INJECTOR**
NADELLOSER INJEKTOR
INJECTEUR SANS AIGUILLE

(30) Priority: 29.06.2020 JP 2020111883
(43) Date of publication of application: 03.05.2023
(73) Proprietor: Daicel Corporation, Osaka 530-0011 (JP)
(72) Inventor: YAMAMOTO, Yuzo, Tokyo 108-8230 (JP); IGA, Hiromitsu, Tokyo 108-8230 (JP)
(74) Representative: Grünecker Patent- und Rechtsanwälte PartG mbB
(86) International application number: PCT/JP2021/022367
(87) International publication number: WO 2022/004334

(56) References cited:
- EP-A1- 3 398 633
- WO-A1-03/051432
- WO-A1-2005/094923
- WO-A1-2005/094923
- WO-A1-2016/031613
- WO-A1-2016/031613

## Description

### Technical Field

The present invention relates to a needleless injector that ejects an injection substance to a target region without using an injection needle.

### Background Art

An example of a device that ejects an injection substance such as a liquid chemical to a target region such as an organism includes a needleless injector using no injection needle, which has been attracting attention in terms of usability and sanitation, and thus has been actively developed recently. The needleless injector requires an actuator that can impart a relatively large amount of energy for ejecting the liquid chemical to the target region, and sending the liquid chemical to the inside of the target region using the energy of the liquid chemical. An example of such an actuator includes a pyro-actuator utilizing energy produced by combustion of an explosive.

Patent Document 1 describes an example of the pyro-actuator, which is applied to a vehicle safety device, and not to a needleless injector. The pyro-actuator features the following mechanism. Specifically, a piston is disposed facing an ignition part of an igniter while positioned on the inner side of a tubular metal case, and the piston is driven by combustion energy produced by the ignition part actuated. The pyro-actuator employs a structure in which the case is covered by a resin housing. This structure secures the heat resistance and pressure resistance of a combustion chamber in the pyro-actuator.

### Citation List

### Patent Document

Patent Document 1: JP 6632437 B Patent document 2: EP 3 398 633 A1

### Summary of Invention

### Technical Problem

The needleless injector requires a user to go through processes of holding the needleless injector and bringing an ejection port of the needleless injector to a point where the liquid chemical or the like needs to be administered. This means that when the needleless injector is large and heavy, the user operability is compromised. In a case where a needleless injector uses, as a pressurization source for the liquid chemical or the like, an igniter that can produce a relatively large amount of energy, the needleless injector needs to have sufficient strength against the pressure produced, to guarantee the safety of the user. However, to increase the strength, generally, it is necessary to use a high strength member such as metal for the injector body or make the injector body itself large. As a result, operability is compromised.

In view of the problem described above, an object of the disclosure of the present application is to provide a technique that can secure sufficient strength of a needleless injector having an igniter as a pressurization source, against pressure produced by the igniter, and can prevent the user operability from being compromised.

### Solution to Problem

The above and other objects of the invention are solved by the needleless injector according to claim 1. Preferred embodiments are claimed in the dependent claims. In order to solve the above problems, the needleless injector of the disclosure of the present application employs a structure in which a tubular reinforcing member formed by a metal member is embedded in an injector body formed by a resin member. With this structure, the injector body is formed of a combination of materials, so that sufficient strength can be secured against pressure produced by combustion of ignition charge in an igniter, and the proportion of the weight of the metal member in the injector body can be reduced as much possible.

Specifically, a needleless injector that ejects an injection substance to a target region without using an injection needle of the disclosure of the present application includes: an injector body formed by a resin member; an igniter that is provided to the injector body, and configured to discharge, into a combustion chamber in the injector body, ejection energy for ejecting the injection substance through combustion of an ignition charge; a housing part including an accommodating space in which the injection substance is accommodated, the housing part defining a flow path that enables the injection substance to be ejected to the target region through an ejection port, and the housing part being provided to the injector body; and a piston that is configured to be moved by the ejection energy in a predetermined path, communicating with the combustion chamber, in the injector body to pressurize the injection substance accommodated in the accommodating space. The injector body is provided with a reinforcing member therein, the reinforcing member being formed by a metal member to be a tubular member, the reinforcing member extending in an axial direction of the injector body while surrounding at least the igniter and the combustion chamber, and the reinforcing member being disposed to be sandwiched between parts of the injector body in a radial direction of the injector body.

In the needleless injector described above, the igniter discharges the ejection energy into the combustion chamber to eject the injection substance to the target region. The piston moves in the predetermined path by being pushed by the ejection energy. Thus, the piston pressurizes the injection substance in the housing part. As a result, the injection substance flows in a flow path of the housing part, to be ejected to the target region. In the needleless injector, the distal end side is a side relatively closer to the ejection port, and the base end side is a side opposite to the distal end side in the longitudinal direction (axial direction) of the needleless injector.

Further, as the intended injection substance ejected from the needleless injector, predetermined substances including a component expected to have effects in the target region or a component expected to exert a predetermined function in the target region can be exemplified. Thus, the intended injection substance may have any physical form, as long as the ejection by the ejection energy as described above can at least be performed. For example, the intended injection substance may be dissolved in liquid, or may be simply mixed with liquid without dissolving therein. As one example, the predetermined substance to be sent includes vaccine for intensifying an antibody, a protein for cosmetic enhancement, a cultured cell for hair regeneration, and the like, and is included in a liquid medium in an ejectable manner. The intended injection substance is formed in this way. Note that the medium is preferably a medium that does not hinder the above-mentioned effect and function of the predetermined substance in a state of being injected into the target region. As another method, the medium may be a medium that exerts the above-mentioned effect and function by acting together with the predetermined substance in the state of being injected into the target region.

The intended injection substance ejected needs to penetrate the surface of the target region such that the intended injection substance is ejected from the needleless injector to the target region to be delivered into the inside thereof. Thus, at an ejection initial state, the intended injection substance needs to be ejected to the target region at a relatively high speed. In view of this point, as an example, the igniter preferably imparts the ejection energy using a combustion product discharged by combustion of an ignition charge. Note that, as the ignition charge, there may be employed any one of an explosive containing zirconium and potassium perchlorate, an explosive containing titanium hydride and potassium perchlorate, an explosive containing titanium and potassium perchlorate, an explosive containing aluminum and potassium perchlorate, an explosive containing aluminum and bismuth oxide, an explosive containing aluminum and molybdenum oxide, an explosive containing aluminum and copper oxide, an explosive containing aluminum and iron oxide, or an explosive composed of a combination of a plurality of the above explosives. As characteristics of the above-mentioned ignition charge, the combustion product is gas at a high temperature but does not include a gas component at a room temperature, hence the combustion product is condensed immediately after the ignition. As a result, the igniter can impart the ejection energy in an extremely short period of time.

In the needleless injector described above, the injector body is formed of a resin material, and the tubular reinforcing member formed of a metal member is provided in the injector body. The injector body is provided with the reinforcing member, with the reinforcing member surrounding at least the igniter and the combustion chamber, that is, with at least the igniter and the combustion chamber contained within the inner side space of the reinforcing member. The combustion chamber is a space in which the igniter itself is disposed, and is a space that receives instantaneous pressure produced by the combustion of the ignition charge. Thus, with the arrangement described above, the needleless injector is configured in such a manner that the pressure produced by the combustion can be favorably received by the reinforcing member featuring relatively high strength. The reinforcing member made of metal is sandwiched between parts of the injector body made of resin, in the radial direction of the injector body. Thus, the injector body formed of a composite material constituted by the resin material and the metal material is disposed against the pressure of the ejection energy spreading in the radial direction of the injector body with the igniter at the center, whereby the strength against the pressure can be effectively improved.

The reinforcing member may have any tubular shape as long as at least the igniter and the combustion chamber can be accommodated in the internal space, and preferably has a cylindrical shape. When the shape is a cylindrical shape, the pressure can be uniformly received, whereby the strength of the injector body can be effectively improved. As an example, the tubular reinforcing member may be formed by a single sheet member, and alternatively, may be formed by a member that has been subjected to predetermined processing such as a mesh member or a perforated metal plate. The reinforcing member may be formed by a single tubular member, or may be formed by any combination of a plurality of tubular members. The reinforcing member may be a linear tubular member with no change in cross-sectional area in the axial direction, and alternatively be a sleeve member with the cross-sectional area changing in the axial direction.

As described above, the needleless injector of the present disclosure employs a structure in which the tubular reinforcing member formed by a metal member is embedded in the injector body formed by the resin member, whereby sufficient strength against the pressure produced by the igniter can be secured, and the proportion of the weight of the metal member in the injector body can be reduced as much as possible. As a result, the size and the weight of the needleless injector can be favorably reduced, and the user operability can be prevented from being compromised. When a metal cylindrical member is used for the reinforcing member, such a cylindrical member can be relatively easily procured and processed. As a result, the manufacturing cost of the needleless injector can be made low.

In the needleless injector described above, the reinforcing member may extend in the axial direction of the injector body, while surrounding at least part of the predetermined path, in addition to the igniter and the combustion chamber. The predetermined path is a space in which the piston moves, and is also a space where the pressure of the energy produced by the combustion of the igniter acts, because the predetermined path communicates with the combustion chamber. Thus, with the reinforcing member extending to surround at least part of the predetermined path as described above, the strength against the pressure can be effectively improved.

In the injector body of the needleless injector, the thickness of part of the injector body forming the combustion chamber may be set to be smaller than the thickness of part of the injector body forming the predetermined path. In this case, the thickness of the first portion of the reinforcing member surrounding the igniter and the combustion chamber may be set to be larger than the thickness of the second portion surrounding the predetermined path. The diameter of the predetermined path may be set to be smaller than the diameter of the igniter and the combustion chamber, to concentrate the energy produced by the combustion in the igniter on the piston for moving the piston efficiently. This may result in the thickness of the part of the injector body forming the combustion chamber being smaller than the thickness of the part of the injector body forming the predetermined path. In such a case, the strength of the former is likely to be lower than that of the latter. In view of this, the thickness of the first portion of the reinforcing member is set to be larger than the thickness of the second portion. Thus, sufficient strength against the pressure produced by the combustion in the igniter is likely to be secured.

In the needleless injector described above, the reinforcing member may be formed by stacking a plurality of metal tubular sub members with different diameters. The number of stacked sub members corresponding to the first portion may be larger than the number of stacked sub members corresponding to the second portion. With the numbers of stacked sub members thus adjusted, the thickness of the first portion of the reinforcing member can be made larger than the thickness of the second portion.

In the needleless injector described so far, the injector body may include a first housing provided with the igniter and a second housing provided with the housing part. In a state where the reinforcing member is disposed on a surface of the first housing or in a state where the reinforcing member is disposed on a surface of the second housing, the first housing and the second housing may be attached to each other to achieve a state where the reinforcing member is sandwiched between the first housing and the second housing. With the injector body thus formed by two housings, the reinforcing member can be easily disposed in the injector body. Any aspect can be employed for attachment between the first housing and the second housing. For example, mating, snap fastening, fastening using bolts, or the like can be used.

In the needleless injector described so far, an additional reinforcing member may be provided that is formed in a tubular shape by a metal member, and forms a surface on which the piston slides, on a surface of the part of the injector body forming the predetermined path. With the additional reinforcing member thus provided, the predetermined path can be favorably protected from the pressure produced by the combustion in the igniter. Furthermore, since the surface on which the piston slides is formed by an additional reinforcing member made of metal, the frictional force acting on the piston can be reduced, whereby the injection substance can be more efficiently ejected.

### Advantageous Effects of Invention

With the disclosure of the present application, sufficient strength of a needleless injector having an igniter as a pressurization source against pressure produced by the igniter can be guaranteed, and the user operability can be prevented from being compromised.

### Brief Description of Drawings

FIG. 1 is a first diagram illustrating a schematic configuration of a needleless injector.
FIG. 2A is a cross-sectional view of the needleless injector illustrated in FIG. 1 taken along line AA.
FIG. 2B is a cross-sectional view of the needleless injector illustrated in FIG. 1 taken along line BB.
FIG. 3 is a second diagram illustrating a schematic configuration of a needleless injector.
FIG. 4 is a third diagram illustrating a schematic configuration of a needleless injector.
FIG. 5 is a fourth diagram illustrating a schematic configuration of a needleless injector.
FIG. 6 is a diagram illustrating attachment of a first housing and a second housing in the needleless injector illustrated in FIG. 4.
FIG. 7 is a fifth diagram illustrating a schematic configuration of a needleless injector.

### Description of Embodiments

With reference to the drawings, a needleless injector 1 (hereinafter, simply referred to as "injector 1") without an injection needle of the disclosure of the present application will be described as an example. Note that each of the configurations, combinations thereof, and the like in each embodiment described in the present application is an example, and additions, omissions, substitutions, and other changes of the configuration may be made as appropriate without departing from the present invention. The present disclosure is not limited by the embodiments and is limited only by the claims.

### First Embodiment

The injector 1 is a needleless injector that injects an ejection solution, which corresponds to an injection substance of the present application, into a target region, using the combustion energy of an explosive. In other words, the injector 1 is a device that injects the ejection solution into the target region without using an injection needle. Note that, in the present embodiment, as terms indicating a relative positional relationship in a longitudinal direction (axial direction) of the injector 1, "distal end side" and "base end side" are used. The "distal end side" indicates a side closer to the distal end of the injector 1 described below, that is, a position closer to an ejection port 77, and the "base end side" indicates a side in an opposite direction toward the "distal end side" in a longitudinal direction of the injector 1, that is, a direction toward an igniter 22 side.

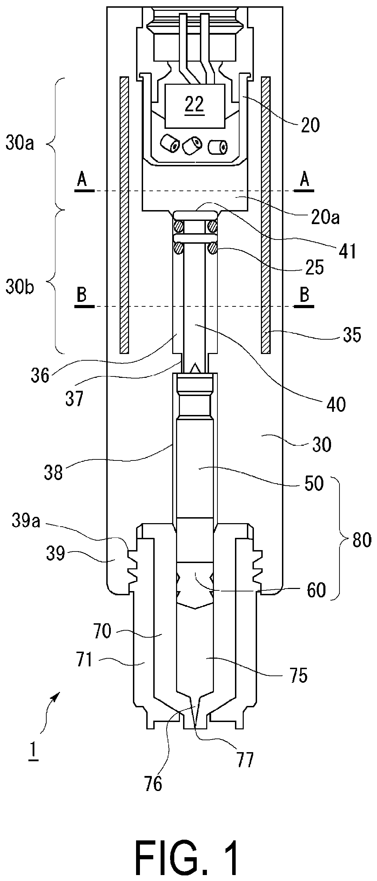

Here, FIG. 1 is a cross-sectional view of the injector 1 illustrating a schematic configuration of the injector 1. The injector 1 includes an igniter 22, an injector body 30, a container 70, a plunger 80, and the like. An accommodating space 75 formed in the container 70 between the plunger 80 and the container 70 included in the injector 1 is filled with ejection solution during a preparation stage before the actuation of the injector 1. Note that, in the following description in the present application, the ejection solution ejected to the target region by the injector 1 is formed of a liquid medium including a predetermined substance, which exerts an effect or a function expected in the target region. In the ejection solution, the predetermined substance may be in a state of being dissolved in liquid being a medium, or may be in a state of being simply mixed instead of being dissolved.

For example, examples of the predetermined substance included in the ejection solution include an organism-derived substance and a substance with a desired bioactivity, which can be ejected to the target region being an organism. For example, examples of the organism-derived substance include DNA, RNA, a nucleic acid, an antibody, and a cell. Examples of the substance with a desired bioactivity include various substances exerting pharmacological or therapeutic effects, which are exemplified by medicines composed of low molecular compounds, proteins, peptides, or the like, a vaccine, an inorganic substance such as metal particles for thermotherapy or radiotherapy, and a carrying body functioning as a carrier. Further, the liquid being the medium of the ejection solution is only required to be a substance suitable for administering the predetermined substance exemplified by those substances to the target region, and may be aqueous or oleaginous, which is not limited. Further, viscosity of the liquid being the medium is not particularly limited as long as the predetermined substance can be ejected by the injector 1.

In the injector 1, the igniter 22 is actuated upon being supplied with ignition current from an external power source, to make the injector 1 inject the ejection solution. The ignition current may be supplied to the igniter 22, under various modes. For example, the power actuating the igniter 22 may be supplied from the outside through a power cable not illustrated. Alternatively, a battery for supplying the power may be provided inside the injector 1.

The igniter 22 is an electric igniter that combusts ignition charge to produce energy for ejection. The igniter 22 has a cap 20 that is ruptured by a combustion product produced when the ignition charge is combusted. The cap 20 is positioned in the combustion chamber 20a, in a state where the igniter 22 is attached to the injector body 30. The actuation of the igniter 22 causes the produced combustion product to rupture the cap 20 and to be discharged into the combustion chamber 20a.

Herein, a combustion energy used in the igniter 22 for the ignition charge is an energy for the injector 1 to eject the ejection solution to the target region. Note that, examples of the ignition charge include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and iron oxide (AFO), or an explosive composed of a combination of a plurality of these explosives. These explosives exhibit characteristics that, although the explosives generate high-temperature and high-pressure plasma during combustion immediately after ignition, when the combustion product condenses at room temperature, the explosives do not contain gaseous components and hence the pressure generated decreases abruptly. An explosive other than these may be used as the ignition charge as long as appropriate ejection of the ejection solution can be performed.

The internal space of the injector body 30 serves as the combustion chamber 20a into which a combustion product is discharged from the igniter 22. The combustion chamber 20a is a cylindrical space having a diameter set to enable the igniter 22 having the cap 20 to fit in the combustion chamber 20a. The injector body 30 is provided with an internal space 36 that communicates with the combustion chamber 20a and that has a cylindrical shape. The internal space 36 is disposed together with O rings 25 serving as a sealing member to make the piston 40 slidable, and the internal space 36 corresponds to a predetermined path of the disclosure of the present application. In a state where the piston 40 is disposed in the internal space 36 before the actuation of the igniter 22, a flange surface 41 on the base end side of the piston 40, which serves as a surface receiving pressure of the combustion product from the igniter 22, is exposed on the combustion chamber 20a side, and the distal end of the piston 40 is accommodated in an internal space 37 communicates with the internal space 36 and has a cylindrical shape on the distal end side of the injector body 30. The internal space 37 has a smaller diameter than the internal space 36.

Then, when the igniter 22 is actuated and the combustion product is discharged into the combustion chamber 20a and thus the pressure therein rises, the flange surface 41 on the base end side of the piston 40 receives the pressure, resulting in the piston 40 moving toward the distal end side. Thus, in the injector 1, the piston 40 is driven with the igniter 22 serving as the pressurization source. The diameter of the radially expanded flange surface 41 of the piston 40 has a larger diameter than the internal space 37, meaning that the movement amount of the piston 40 pressing the plunger 80 is limited. The piston 40 may be formed of a resin, and in such a case, metal may be used together for a part where heat resistance and pressure resistance are required.

Additionally, as an alternative mechanism to adjust the pressure applied to the piston 40, the combustion chamber 20a toward which the cap 20 of the igniter 22 faces may be further provided with a gas generating agent that is burned by the combustion product from the igniter 22 to produce gas. The agent may be disposed, for example, at a location that may be exposed to the combustion product from the igniter 22. Further, as another method, the gas generating agent may be disposed in the igniter 22 as disclosed in WO 01-031282, JP 2003-25950 A, and the like. As one example of the gas generating agent, there may be exemplified a single base smokeless explosive formed of 98 mass% of nitrocellulose, 0.8 mass% of diphenylamine, and 1.2 mass% of potassium sulfate. Further, various types of gas generating agents used in a gas generator for an air bag and a gas generator for a seat belt pretensioner may be used. A combustion completion time period of the gas generating agent can be changed by adjusting a dimension, a size, a shape, and particularly, a surface shape of the gas generating agent at the time of being disposed in the combustion chamber 20a or the like. With this, the pressure applied to the piston 40 can be adjusted to a desired pressure.

The injector body 30 is a component to be the main body of the injector 1, for attaching the igniter 22, the plunger 80, the container 70, and the like as illustrated in FIG. 1. Here, a known resin member such as nylon 6-12, polyarylate, polybutylene terephthalate, polyphenylene sulphide, a liquid crystal polymer, or the like may be used for the injector body 30 for example. Further, a filler such as glass fibers and glass filler may be contained in those resins. 20 to 80 mass% of glass fibers may be contained in polybutylene terephthalate, 20 to 80 mass% of glass fibers may be contained in polyphenylene sulphide, or 20 to 80 mass% of minerals may be contained in a liquid crystal polymer.

In the injector body 30, an internal space 38 is formed to communicate with the internal space 37. The internal space 38 is a region in which the plunger 80 is generally disposed as illustrated in FIG. 1, and is a hollow region formed in a cylindrical shape extending along the axial direction of the injector body 30. The internal space 38 has a diameter that is larger than that of the internal space 37, and enables the plunger 80 to slide therein. The plunger 80 is a member that pressurizes the ejection solution using the energy received from the piston 40, and has, on its base end side, a plunger rod 50 that receives force from the piston 40, and has, on its distal end side, a stopper portion 60 that is formed by an elastic member such as rubber and that presses the ejection solution. The injector body 30 may have a through hole formed through a side outer surface to reach the internal space 38. Through the through hole, the user can check the status (such as whether the injector 1 is before or after actuation, for example) of the plunger 80 in the injector 1 from the outside.

In an inner wall of a distal end portion 39 of the injector body 30, a female thread portion 39a for attaching the container 70 is formed. The container 70 is attached to the injector body 30 via the female thread portion 39a, using a container holder 71 described below. The container 70 is a member containing an ejection solution to be pressurized by the plunger 80, is a member that defines a flow path 76 for ejecting the pressurized ejection solution to the target region through the ejection port, and corresponds to the housing part. In view of this, a resin material (a resin material of the same type as the injector body 30 for example) may be used for forming the container 70.

The container 70 includes an accommodating space 75 that is formed to enable the stopper portion 60 of the plunger 80 to be movable therein, and to accommodate the ejection solution, and a flow path 76 connecting the accommodating space 75 to the ejection port 77 facing the outside of the container 70. The container 70 itself thus formed does not have a male thread portion mating with the female thread portion 39a of the injector body 30. Thus, the attachment of the container 70 to the injector body 30 is implemented using the container holder 71. The container holder 71 accommodates the container 70 with the ejection port 77 of the container 70 exposed through a distal end side opening of the container holder 71. At this time, the container 70 is in a state in which the accommodating space 75 is filled with the injection substance and the plunger 80 is loaded. In a state where the container 70 is accommodated in the container holder 71, the male thread of the container holder 71 and the female thread portion 39a of the injector body 30 are mated until the base end side end surface of the container 70 comes into contact with the end surface of the injector body 30. As a result, the container 70 is sandwiched between the container holder 71 and the injector body 30, with the fastening force produced by the mating. In this state, the plunger 80 is in a state of being located inside both the accommodating space 75 in the container 70 and the internal space 38 in the injector body 30.

The state illustrated in FIG. 1 is a state where the container 70 is thus attached to the injector body 30 by the container holder 71. In this state, when the igniter 22 is actuated, the combustion product produced therein pressurizes the piston 40, and the plunger 80 slides in the internal space 38. As a result, the ejection solution accommodated in the accommodating space 75 is pressurized to be ejected from the ejection port 77 through the flow path 76. The flow path 76 provided in the container 70 has a diameter smaller than the inner diameter of the accommodating space 75. With this configuration, the ejection solution that has been applied with a high pressure is ejected to the outside through the ejection port 77.

Note that the profile on the distal end side of the stopper portion 60 of the plunger 80 is shaped to generally match the profile of the inner surface near a portion where the accommodating space 75 and the flow path 76 are connected to each other (the deepest part of the accommodating space 75). With this configuration, a smallest possible gap can be formed between the stopper portion 60 and the inner surface of the container 70 when the plunger 80 slides for ejecting the ejection solution and reaches the deepest part of the accommodating space 75, whereby the ejection solution can be prevented from wastefully remaining in the accommodating space 75. However, the shape of the stopper portion 60 is not limited to a particular shape as long as desired effects can be obtained with the injector 1 according to the present embodiment.

The igniter 22 is used in the injector 1 for the pressurization source for ejecting the ejection solution. Thus, when the igniter 22 is actuated, a relatively large pressure acts instantaneously on the inner wall of the combustion chamber 20a that is exposed to the combustion product and the inner wall of the internal space 36 in which the piston 40 moves. In the injector body 30, the piston 40 is movable as viewed in the axial direction of the injector 1. However, since there is no member movable in the radial direction thereof, when the pressure rises, a corresponding amount of stress acts on the inside of the injector body 30. To suppress deformation of or damage to the injector body 30, a tubular reinforcing member 35 formed by a metal member is disposed in the injector body 30. Examples of the metal material that can be used include aluminum, iron, copper, steel, or an alloy of these. In the present disclosure, the reinforcing member 35 has a cylindrical shape, but other tubular shapes may be employed.

As illustrated in FIG. 1, in the injector body 30, a portion where the reinforcing member 35 surrounds the igniter 22 and the combustion chamber 20a, that is, a portion including the igniter 22 and the combustion chamber 20a disposed on the inner side of the reinforcing member 35 in a cross-sectional view of the injector body 30 taken along the radial direction is defined as a first portion 30a. In the injector body 30, a portion where the reinforcing member 35 surrounds the internal space 36, that is a portion including the internal space 36 positioned on the inner side of the reinforcing member 35 in the cross-sectional view of the injector body 30 taken along the radial direction is defined as a second portion 30b. The reinforcing member 35 is formed to extend in both the first portion 30a and the second portion 30b, along the axial direction of the injector 1.

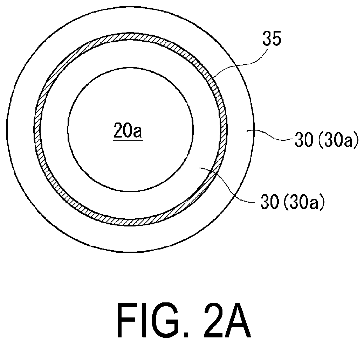

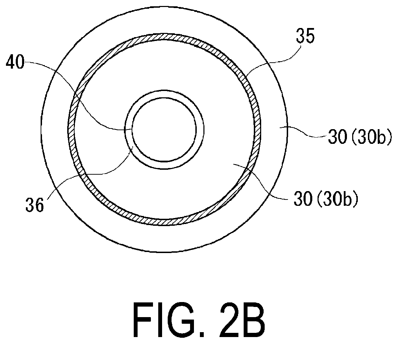

FIG. 2A is a cross-sectional view of the injector 1 taken along line AA in the first portion 30a. FIG. 2B is a cross-sectional view of the injector 1 taken along line BB in the second portion 30b. As can be seen in FIGS. 2A and 2B, the injector 1 is configured in such a manner that in the first portion 30a and the second portion 30b where a large pressure, produced by the combustion of the explosive in the igniter 22, may be applied on the inner wall in the radial direction of the injector body 30, the reinforcing member 35 made of metal can favorably receive the pressure. Furthermore, in the radial direction of the injector body 30, the reinforcing member 35 made of metal is sandwiched by parts of the injector body 30 made of resin. Thus, the injector body 30 formed by a combination of materials including the resin material and the metal material is disposed against the pressure, whereby strength against the pressure can be effectively improved. Since the reinforcing member 35 has a cylindrical shape, the pressure can be uniformly received, whereby the strength of the injector body 30 can be effectively improved.

Furthermore, when a cylindrical metal member is used as the reinforcing member 35, a "metal pipe" that can be easily procured and processed is generally used as the member. Thus, the reinforcing member 35 can be easily formed by preparing a ready-made metal pipe having a diameter dimension with which the igniter 22 and the combustion chamber 20a can be accommodated, and cutting the metal pipe in accordance with the lengths of the first portion 30a and the second portion 30b in the axial direction. Then, in a manufacturing step for the injector 1, the injector body 30 illustrated in FIG. 1 can be formed through insert molding with a predetermined resin material injected in a mold, in a state where the reinforcing member 35 is disposed at a predetermined position in the mold for forming the injector body 30. In this process, the igniter 22 may be also formed by insert molding.

Thus, the injector 1 illustrated in FIG. 1 can effectively protect the injector body 30 against pressure produced when the igniter 22 is actuated by adopting a configuration in which a metal reinforcing member 35 is embedded in the injector body 30 made of resin. In particular, since the injector body 30 can have the strength effectively improved with the resin material and the metal material combined, the proportion of the metal material in the injector body 30 can be reduced, whereby the injector 1 can have a smaller size and a lighter weight. All things considered; the user operability can be prevented from being compromised. Furthermore, the injector 1 can be easily manufactured as described above, whereby an extremely large advantage in terms of manufacturing cost can also be achieved.

### Variation

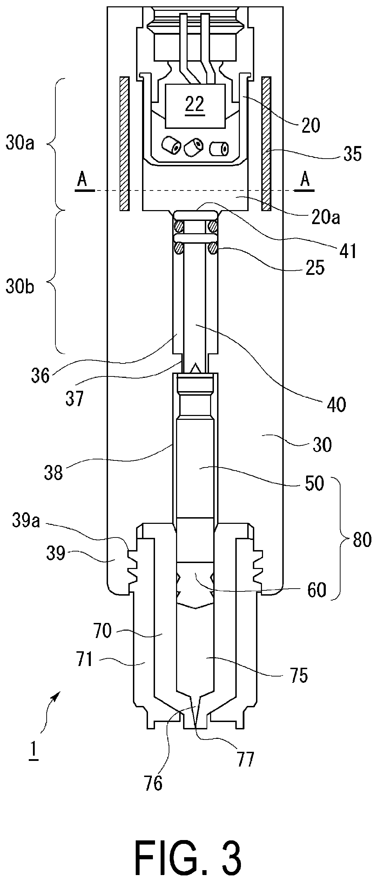

A variation of the present embodiment related to the reinforcing member 35 in the injector 1 of the disclosure of the present application will be described based on FIG. 3. In the injector 1 illustrated in FIG. 3, the reinforcing member 35 extends only in the first portion 30a in the injector body 30. The thickness of the injector body 30 in the radial direction is larger in the second portion 30b than in the first portion 30a. In the second portion 30b, the piston 40 moves in the internal space 36, meaning that the rate of increase in pressure in the internal space 36 may be lower than that in the combustion chamber 20a at the initial point of actuation of the igniter 22. Thus, the strength of the second portion 30b against pressure can be relatively easily secured. If the strength of the second portion 30b can be sufficiently secured, the reinforcing member 35 may have a length set to extend only in the first portion 30a as illustrated in FIG. 3.

### Second Embodiment

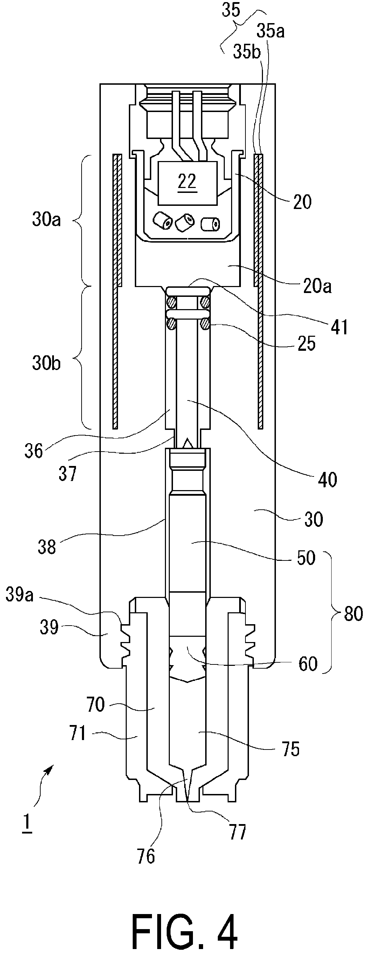

Next, a second embodiment of the injector 1 of the present disclosure will be described based on FIG. 4. FIG. 4 is a diagram illustrating a schematic configuration of the injector 1 as in FIG. 1. Of the configurations illustrated in FIG. 4, those that are substantially the same as the counterparts illustrated in FIG. 1 are denoted by the same reference numerals, and the detailed description thereof will be omitted.

The reinforcing member 35 of the injector 1 illustrated in FIG. 4 is formed with two sub members 35a and 35b stacked. The sub members 35a and 35b are each a cylindrical member formed of a metal material (for example, aluminum, iron, copper, steel, or an alloy of these). The sub member 35a has an inner diameter that is equal to or larger than an outer diameter of the sub member 35b, and thus the sub member 35b can be accommodated in the internal space of the sub member 35a in a stacked manner. The sub member 35a has a length in the axial direction set in such a manner that the sub member 35a extends in the first portion 30a and the second portion 30b of the injector body 30. The sub member 35b has a length in the axial direction set in such a manner that the sub member 35b extends only in the first portion 30a of the injector body 30.

As described above, the strength of the second portion 30b against the pressure produced when the igniter 22 is actuated, can be easily secured, compared with the first portion 30a. Thus, the minimum required reinforcement level differs between the first portion 30a and the second portion 30b of the injector body 30. With the reinforcing member 35 formed with sub members having different diameters stacked so that required strength can be provided for each portion as described above, the amount of metal of the reinforcing member 35 required for reinforcing the entire injector 1 can be adjusted to be of a minimum required amount. As a result, the size and weight of the injector 1 can be reduced. Since the reinforcing member 35 can be formed by stacking the sub members 35a and 35b that are tubular metal pipes, the reinforcing member 35 can be easily manufactured, whereby the manufacturing cost of the injector 1 can be made low.

In the example illustrated in FIG. 4, the number of stacked sub members is different between the first portion 30a and the second portion 30b. For example, when there is a further portion to be reinforced in the first portion 30a, a further sub member with a different diameter may be prepared to increase the number of stacked sub members, to improve the strength of the portion. The same applies to the second portion 30b.

### Third Embodiment

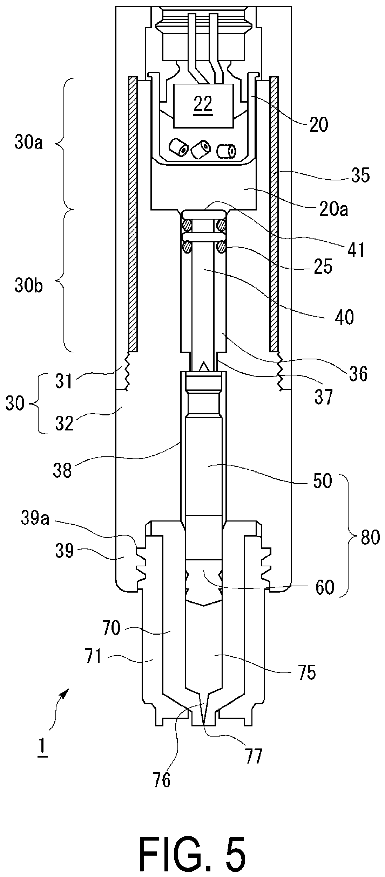

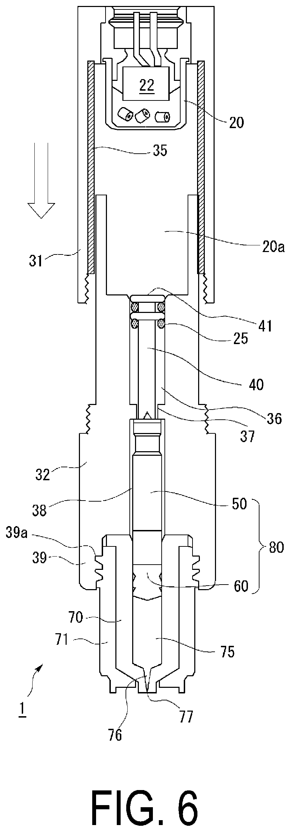

Next, a third embodiment of the injector 1 of the present disclosure will be described based on FIG. 5 and FIG. 6. FIG. 5 is a diagram illustrating a schematic configuration of the injector 1 as in FIG. 1. Of the configurations illustrated in FIG. 5, those that are substantially the same as the counterparts illustrated in FIG. 1 are denoted by the same reference numerals, and the detailed description thereof will be omitted. FIG. 6 illustrates a procedure for assembling the injector 1 illustrated in FIG. 5.

The reinforcing member 35 in the injector 1 illustrated in FIG. 5 and FIG. 6 has the same configuration as the reinforcing member 35 in the injector 1 illustrated in FIG. 1 in that it is a metal tubular member having a length set to extend in the first portion 30a and the second portion 30b in the injector body 30. As illustrated in FIG. 6, the injector body 30 is formed by mating and integrating a first housing 31 provided with the igniter 22 and a second housing 32 to which the container 70 is attached. The first housing 31 has a tubular shape of a female type, and has an internal space with a bottom surface on which the igniter 22 is positioned. The second housing 32 has a shape of a male type that can be fitted in the internal space of the first housing 31. A space formed to serve as the combustion chamber 20a is formed on the base end side of the second housing 32 (the side opposite to the first housing 31). With the housings combined, the injector 1 has a structure illustrated in FIG. 5. The injector 1 illustrated in FIG. 5 has substantially the same structure, except that the injector body 30 is formed by the two housings.

In the injector 1 having such an assembly structure, as illustrated in FIG. 6, in a state where the reinforcing member 35, which is tubular, is disposed on the surface of the internal space of the first housing 31, the second housing 32 is fitted in and mated with, and thus attached to the first housing 31. Thus, a gap in which the reinforcing member 35 can be disposed is formed between the first housing 31 and the second housing 32. As a result of attaching the housings, a state in which the reinforcing member 35 is sandwiched between the first housing 31 and the second housing 32 is achieved. This state is substantially the same as the arrangement of the reinforcing members 35 illustrated in FIG. 1. Alternatively, the injector 1 as illustrated in FIG. 5 may be formed with the first housing 31 fitted in, mated with, and thus attached to the second housing 32 in a state where the tubular reinforcing member 35 is disposed on the outer surface of the second housing 32 having the male type shape as described above.

According to the present embodiment, the reinforcing members 35 can be disposed in the injector body 30 by a step of attaching the two housings to each other. The embodiment illustrated in FIG. 1 requires the reinforcing member 35 to be disposed at the same timing as the molding of the injector body 30 by the resin material, and thus requires insert molding. With the present embodiment, the reinforcing member 35 can be similarly disposed without performing the insert molding. Thus, the injector 1 can be manufactured easily, whereby the manufacturing cost thereof can be reduced.

### <Fourth Embodiment>

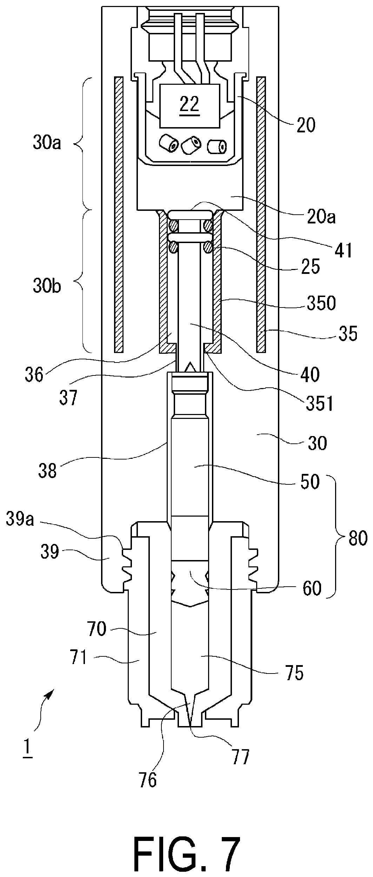

Next, a fourth embodiment of the injector 1 of the present disclosure will be described based on FIG. 7. FIG. 7 is a diagram illustrating a schematic configuration of the injector 1 as in FIG. 1. Of the configurations illustrated in FIG. 7, those that are substantially the same as the counterparts illustrated in FIG. 1 are denoted by the same reference numerals, and the detailed description thereof will be omitted.

The reinforcing member 35 in the injector 1 illustrated in FIG. 7 has the same configuration as the reinforcing member 35 in the injector 1 illustrated in FIG. 1 in that it is a metal tubular member having a length set to extend in the first portion 30a and the second portion 30b in the injector body 30. In the injector 1 illustrated in FIG. 7, a reinforcing member 350 forming a surface on which the piston 40 slides and serving as an additional reinforcing member is further provided on the surface of the injector body 30 forming the internal space 36. The reinforcing member 350 is formed of the same metal member as the reinforcing member 35. The reinforcing member 350 is a tubular member having a space to serve as the internal space 36 defined on the inner side, and has an opening 351 through which the piston 40 can pass formed on the distal end side. The opening 351 communicates with the internal space 37. The opening 351 has a smaller diameter than the flange surface 41 of the piston 40, and thus the opening 351 does not hinder the movement of the piston 40.

With the additional reinforcing member 350 thus provided, the strength of the second portion 30b against the pressure produced when the igniter 22 is actuated can be improved, and the surface on which the piston 40 slides is formed by a metal surface so that the frictional force acting on the piston 40 can be reduced, whereby the ejection solution can be ejected more efficiently. The movement of the piston 40 is received and stopped by the reinforcing member 350 made of metal. Thus, the impact produced in this process is received by the metal member, whereby the injector body 30 can be prevented from deforming or being damaged.

Each aspect disclosed in the specification of the present application can be combined with any other feature disclosed herein within the scope of the claims. The scope of protection is only defined by the appended claims.

### Reference Signs List

1 Injector (needleless injector)
22 Igniter
30 Injector body
30a First portion
30b Second portion
31 First housing
32 Second housing
35 Reinforcing member
350 Reinforcing member
351 Opening
40 Piston
70 Container
71 Container holder
75 Accommodating space
76 Flow path
77 Ejection port
80 Plunger

## Claims

1. A needleless injector (1) that ejects an injection substance to a target region without using an injection needle, the needleless injector (1) comprising:
an injector body (30) formed by a resin member;
an igniter (22) that is provided to the injector body (30), and configured to discharge, into a combustion chamber (20a) in the injector body (30), ejection energy for ejecting the injection substance through combustion of ignition charge;
a housing part including an accommodating space (75) in which the injection substance is accommodated, the housing part defining a flow path (76) to allow the injection substance to be ejected to the target region through an ejection port (77), and the housing part being provided to the injector body (30); and
a piston (40) that is configured to be moved by the ejection energy in a predetermined path, communicating with the combustion chamber (20a), in the injector body (30) to pressurize the injection substance accommodated in the accommodating space (75), wherein
the injector body (30) is provided with a reinforcing member (35) therein, the reinforcing member (35) being formed by a metal member to be a tubular member, the reinforcing member (35) extending in an axial direction of the injector body (30) while surrounding at least the igniter (22) and the combustion chamber (20a), and the reinforcing member (35) being disposed to be sandwiched between parts of the injector body (30) in a radial direction of the injector body (30).

2. The needleless injector (1) according to claim 1, wherein the reinforcing member (35) extends in the axial direction of the injector body (30), while surrounding at least part of the predetermined path, in addition to the igniter (22) and the combustion chamber (20a).

3. The needleless injector (1) according to claim 2, wherein
a thickness of part of the injector body (30) forming the combustion chamber (20a) is set to be smaller than a thickness of part of the injector body (30) forming the predetermined path, and
a thickness of a first portion (30a) of the reinforcing member (35) surrounding the igniter (22) and the combustion chamber (20a) is set to be larger than a thickness of a second portion (30b) of the reinforcing member (35) surrounding the predetermined path.

4. The needleless injector (1) according to claim 3, wherein
the reinforcing member (35) is formed by stacking a plurality of tubular sub members (35a, 35b) made of metal and having different diameters, and
a number of the stacked sub members (35a, 35b) corresponding to the first portion (30a) is larger than a number of the stacked sub members (35a), corresponding to the second portion (30b).

5. The needleless injector (1) according to any one of claims 1 to 4, wherein
the injector body (30) includes a first housing (31) provided with the igniter (22) and a second housing (32) provided with the housing part, and
in a state where the reinforcing member (35) is disposed on a surface of the first housing (31) or in a state where the reinforcing member (35) is disposed on a surface of the second housing (32), the first housing (31) and the second housing (32) are attached to each other to achieve a state where the reinforcing member (35) is sandwiched between the first housing (31) and the second housing (32).

6. The needleless injector (1) according to any one of claims 1 to 5, wherein an additional reinforcing member (350) is provided that is formed by a metal member to have a tubular shape, and forms a surface on which the piston (40) slides, on a surface of the part of the injector body (30) forming the predetermined path.

## Patentansprüche

1. Ein nadelloser Injektor (1), der eine Injektionssubstanz in einen Zielbereich ausstößt, ohne eine Injektionsnadel zu verwenden, wobei der nadellose Injektor (1) Folgendes aufweist:
einen Injektorkörper (30), der durch ein Kunststoffelement gebildet wird;
einen Zünder (22), der an dem Injektorkörper (30) vorgesehen ist und so konfiguriert ist, dass er in eine Verbrennungskammer (20a) in dem Injektorkörper (30) Ausstoßenergie zum Ausstoßen der Injektionssubstanz durch Verbrennung der Zündladung abgibt;
ein Gehäuseteil, das einen Unterbringungsraum (75) aufweist, in dem die Injektionssubstanz untergebracht ist, wobei das Gehäuseteil einen Strömungsweg (76) definiert, um zu ermöglichen, dass die Injektionssubstanz durch eine Ausstoßöffnung (77) in den Zielbereich ausgestoßen wird, und das Gehäuseteil an dem Injektorkörper (30) vorgesehen ist; und
einen Kolben (40), der so konfiguriert ist, dass er durch die Ausstoßenergie auf einem vorbestimmten Weg, der mit der Verbrennungskammer (20a) in Verbindung steht, in dem Injektorkörper (30) bewegt wird, um die in dem Unterbringungsraum (75) untergebrachte Injektionssubstanz unter Druck zu setzen, wobei
der Injektorkörper (30) mit einem Verstärkungselement (35) darin versehen ist, wobei das Verstärkungselement (35) durch ein Metallelement gebildet ist, um ein rohrförmiges Element zu sein, wobei sich das Verstärkungselement (35) in einer axialen Richtung des Injektorkörpers (30) erstreckt, während es zumindest den Zünder (22) und die Verbrennungskammer (20a) umgibt, und wobei das Verstärkungselement (35) angeordnet ist, um zwischen Teilen des Injektorkörpers (30) in einer radialen Richtung des Injektorkörpers (30) eingeschlossen zu werden.

2. Der nadellose Injektor (1) nach Anspruch 1, wobei sich das Verstärkungselement (35) in der axialen Richtung des Injektorkörpers (30) erstreckt, während es zumindest einen Teil des vorbestimmten Weges umgibt, zusätzlich zu dem Zünder (22) und der Verbrennungskammer (20a).

3. Der nadellose Injektor (1) nach Anspruch 2, wobei
eine Dicke eines Teils des Injektorkörpers (30), der die Verbrennungskammer (20a) bildet, so eingestellt ist, dass sie kleiner ist als eine Dicke eines Teils des Injektorkörpers (30), der den vorbestimmten Weg bildet, und
eine Dicke eines ersten Abschnitts (30a) des Verstärkungselements (35), das den Zünder (22) und die Brennkammer (20a) umgibt, so eingestellt ist, dass sie größer ist als eine Dicke eines zweiten Abschnitts (30b) des Verstärkungselements (35), das die vorbestimmte Bahn umgibt.

4. Der nadellose Injektor (1) nach Anspruch 3, wobei
das Verstärkungselement (35) durch Stapeln einer Vielzahl von rohrförmigen Unterelementen (35a, 35b) gebildet wird, die aus Metall gefertigt sind und unterschiedliche Durchmesser aufweisen, und
eine Anzahl der gestapelten Unterelemente (35a, 35b), die dem ersten Abschnitt (30a) entspricht, größer ist als eine Anzahl der gestapelten Unterelemente (35a), die dem zweiten Abschnitt (30b) entspricht.

5. Der nadellose Injektor (1) nach einem der Ansprüche 1 bis 4, wobei
der Injektorkörper (30) ein erstes Gehäuse (31), das mit dem Zünder (22) versehen ist, und ein zweites Gehäuse (32), das mit dem Gehäuseteil versehen ist, aufweist, und
in einem Zustand, in dem das Verstärkungselement (35) auf einer Oberfläche des ersten Gehäuses (31) angeordnet ist, oder in einem Zustand, in dem das Verstärkungselement (35) auf einer Oberfläche des zweiten Gehäuses (32) angeordnet ist, das erste Gehäuse (31) und das zweite Gehäuse (32) aneinander befestigt sind, um einen Zustand zu erreichen, in dem das Verstärkungselement (35) zwischen dem ersten Gehäuse (31) und dem zweiten Gehäuse (32) eingeschlossen ist.

6. Der nadellose Injektor (1) nach einem der Ansprüche 1 bis 5, wobei ein zusätzliches Verstärkungselement (350) vorgesehen ist, das durch ein Metallelement gebildet wird, um eine rohrförmige Form zu haben, und eine Oberfläche bildet, auf der der Kolben (40) auf einer Oberfläche des Teils des Injektorkörpers (30) gleitet, der den vorbestimmten Weg bildet.

## Revendications

1. Injecteur sans aiguille (1) qui éjecte une substance d'injection à une région cible sans utiliser d'aiguille d'injection, l'injecteur sans aiguille (1) comprenant :
un corps d'injecteur (30) formé par un élément de résine ;
un dispositif d'allumage (22) qui est prévu au niveau du corps d'injecteur (30), et configuré pour évacuer, dans une chambre de combustion (20a) dans le corps d'injecteur (30), de l'énergie d'éjection pour éjecter la substance d'injection à travers une combustion de charge d'allumage ;
une pièce de logement incluant un espace d'accueil (75) dans lequel la substance d'injection est logée, la pièce de logement définissant un trajet d'écoulement (76) pour permettre à la substance d'injection d'être éjectée vers la région cible à travers un orifice d'éjection (77), et la pièce de logement étant prévue pour le corps d'injecteur (30) ; et
un piston (40) qui est configuré pour être déplacé par l'énergie d'éjection dans un trajet prédéterminé, communiquant avec la chambre de combustion (20a), dans le corps d'injecteur (30) pour placer sous pression la substance d'injection logée dans l'espace d'accueil (75), dans lequel
le corps d'injecteur (30) est pourvu d'un élément de renfort (35) dans lequel, l'élément de renfort (35) étant formé par un élément métallique pour être un élément tubulaire, l'élément de renfort (35) s'étendant dans une direction axiale du corps d'injecteur (30) tout en entourant au moins le dispositif d'allumage (22) et la chambre de combustion (20a), et l'élément de renfort (35) étant disposé pour être pris en sandwich entre des parties du corps d'injecteur (30) dans une direction radiale du corps d'injecteur (30).

2. Injecteur sans aiguille (1) selon la revendication 1, dans lequel l'élément de renfort (35) s'étend dans la direction axiale du corps d'injecteur (30), tout en entourant au moins une partie du trajet prédéterminé, en plus du dispositif d'allumage (22) et de la chambre de combustion (20a).

3. Injecteur sans aiguille (1) selon la revendication 2, dans lequel
une épaisseur d'une partie du corps d'injecteur (30) formant la chambre de combustion (20a) est définie pour être inférieure à une épaisseur d'une partie du corps d'injecteur (30) formant le trajet prédéterminé, et une épaisseur d'une première portion (30a) de l'élément de renfort (35) entourant le dispositif d'allumage (22) et la chambre de combustion (20a) est définie pour être plus grande qu'une épaisseur d'une seconde portion (30b) de l'élément de renfort (35) entourant le trajet prédéterminé.

4. Injecteur sans aiguille (1) selon la revendication 3, dans lequel
l'élément de renfort (35) est formé par l'empilement d'une pluralité de sous-éléments (35a, 35b) tubulaires constitués de métal et ayant différents diamètres, et
un nombre de sous-éléments (35a, 35b) empilés correspondant à la première portion (30a) est plus grand qu'un nombre des sous-éléments (35a) empilés, correspondant à la seconde portion (30b).

5. Injecteur sans aiguille (1) selon l'une quelconque des revendications 1 à 4, dans lequel
le corps d'injecteur (30) inclut un premier logement (31) prévu avec le dispositif d'allumage (22) et un second logement (32) pourvu de la pièce de logement, et
dans un état où l'élément de renfort (35) est disposé sur une surface du premier logement (31) ou dans un état où l'élément de renfort (35) est disposé sur une surface du second logement (32), le premier logement (31) et le second logement (32) sont fixés l'un à l'autre pour atteindre un état dans lequel l'élément de renfort (35) est pris en sandwich entre le premier logement (31) et le second logement (32).

6. Injecteur sans aiguille (1) selon l'une quelconque des revendications 1 à 5, dans lequel un élément de renfort additionnel (350) est prévu qui est formé d'un élément métallique pour présenter une forme tubulaire, et forme une surface sur laquelle le piston (40) coulisse, sur une surface de la pièce du corps d'injecteur (30) formant le trajet prédéterminé.
